(19)

Europäisches
Patentamt
European
Patent Office
Office européen
des brevets

(11) **EP 2 784 093 B1**

(12) **EUROPEAN PATENT SPECIFICATION**

(45) Date of publication and mention
of the grant of the patent:
**09.10.2019 Bulletin 2019/41**

(51) Int Cl.:
*A61K 47/60* (2017.01)      *A61K 38/10* (2006.01)
*C07K 17/08* (2006.01)      *A61P 35/00* (2006.01)

(21) Application number: **12851929.5**

(22) Date of filing: **17.11.2012**

(86) International application number:
**PCT/CN2012/084788**

(87) International publication number:
**WO 2013/075600 (30.05.2013 Gazette 2013/22)**

(54) **POLYETHYLENE GLYCOL-MODIFIED INTEGRIN BLOCKER HM-3 AND USE THEREOF**

MIT POLYETHYLENGLYKOL MODIFIZIERTER INTEGRINBLOCKER HM-3 UND VERWENDUNG
DAVON

BLOQUEUR DE TYPE HM-3 D'INTÉGRINE MODIFIÉ PAR DU POLYÉTHYLÈNEGLYCOL ET SON
UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.11.2011 CN 201110370529**

(43) Date of publication of application:
**01.10.2014 Bulletin 2014/40**

(73) Proprietor: **Xu, Hanmei
Nanjing, Jiangsu 211198 (CN)**

(72) Inventors:
• **CHANG, Haimin
Nanjing
Jiangsu 211198 (CN)**
• **KANG, Zhian
Nanjing
Jiangsu 210009 (CN)**
• **XU, Hanmei
Nanjing, Jiangsu 211198 (CN)**

(74) Representative: **Engelhard, Markus
Boehmert & Boehmert
Anwaltspartnerschaft mbB
Pettenkoferstrasse 22
80336 München (DE)**

(56) References cited:
**CN-A- 102 145 161      CN-A- 102 145 161
CN-A- 102 178 656      CN-A- 102 205 110
CN-A- 102 417 540**

• **B. ZHU ET AL: "Site-specific modification of
anti-angiogenesis peptide HM-3 by polyethylene
glycol molecular weight of 20 kDa", JOURNAL OF
BIOCHEMISTRY, vol. 148, no. 3, 1 September
2010 (2010-09-01), pages 341-347, XP055197298,
ISSN: 0021-924X, DOI: 10.1093/jb/mvq070**
• **ZHENDONG LIU ET AL: "In Vivo Anti-Tumor
Activity of Polypeptide HM-3 Modified by
Different Polyethylene Glycols (PEG)",
INTERNATIONAL JOURNAL OF MOLECULAR
SCIENCES, vol. 12, no. 12, 19 April 2011
(2011-04-19), pages 2650-2663, XP055197271,
DOI: 10.3390/ijms12042650**
• **LIU, ZHENDONG ET AL.: 'Tissue Distribution and
Excretion of PEGylation Polypeptide HM-3 in
Rats' PHARMACEUTICAL BIOTECHNOLOGY
October 2011, pages 416 - 420, XP008173919**
• **KANG ZHOU ET AL: "Studies of Poly(ethylene
glycol) Modification of HM-3 Polypeptides",
BIOCONJUGATE CHEMISTRY, vol. 20, no. 5, 20
May 2009 (2009-05-20), pages 932-936,
XP055429939, ISSN: 1043-1802, DOI:
10.1021/bc900070r**
• **Hanmei Xu ET AL: "RGD-Modified Angiogenesis
Inhibitor HM-3 Dose: Dual Function during
Cancer Treatment", Bioconjugate Chemistry, vol.
22, no. 7, 20 July 2011 (2011-07-20), pages
1386-1393, XP055429973, ISSN: 1043-1802, DOI:
10.1021/bc2000929**

**Description**

**Technical field**

The present invention involves the pharmaceutical field, which is related to an integrin inhibitor being able to inhibit angiogenesis of tumors, having the integrin affinity and binding ability, wherein said inhibitor is a polypeptide modified by polyethylene glycol and said polyethylene glycol modified polypeptide can be used to treat solid tumors.

**Background technology**

According to research, the growth of solid tumors relies on angiogenesis, which can not only provide nutrients and oxygen and excrete metabolites for tumors, but also the pathway of distant metastasis. Therefore, blocking angiogenesis is a method that can prevent tumor growth and metastasis. And thus this theory stimulates extensive research on the pro-angiogenic molecules and anti-angiogenic molecules.

Compared with the traditional medicine of tumors, the advantages of angiogenesis inhibitor are as follows: (1) Selectively affect vascular endothelial cells, and have a relatively lower systemic toxic side effects. (2) The target cells are vascular endothelial cells, which is easy for medicine to reach and take effect. (3) There is no or rare vascular endothelial cell mutant occurring and fewer drug resistance, and therefore it is possible to have a long-term medication on the patients. (4) It can be applied together with chemotherapy methods and reduce the toxicity of the latter.

Currently, the integrin antagonists exploited internationally has entered phase II clinical experiment, whereas there have been no similar products entering Chinese market. So it is necessary to develop such kind of drugs having our own intellectual property rights. Several antagonists are introduced in patent ZL2005100403785 which is related to high efficient methods and applications of polypeptide for angiogenesis inhibition. One of them is integrin antagonist polypeptide, and its sequence is Ile-Val-Arg-Arg-Ala-Asp-Arg-Ala-Ala-Val-Pro-Gly-Gly-Gly-Gly-Arg-Gly-Asp, which contains integrin ligand sequences (Gly-Gly-Gly-Gly-Arg-Gly-Asp) and angiogenesis inhibition sequence (Ile-Val-Arg-Arg-Ala-Asp-Arg-Ala-Ala-Val-Pro). Integrin ligand sequences contain RGD sequences (Arg-Gly-Asp). Integrin antagonist polypeptide sequence can effectively bind integrin subtypes of tumor-specific expression and inhibit angiogenesis of tumors because of its angiogenesis inhibition sequence, eventually come to the results of preventing growth and metastasis of tumors. Previous research demonstrated that its targets are integrin $\alpha v\beta 3$ and $\alpha 5\beta 1$, and $\alpha v\beta 3$ is the prime target. The polypeptide was testified to have better anti-tumor effect via repeated activity evaluation in vivo and vitro. It can significantly inhibit endothelial cell migration and tumor angiogenesis, thereby inhibit tumor growth. However, the half-life of the polypeptide is short and thus clinically, this medicine is administered through intravenous infusion every day and hence brings some pains to the patients.

CN 102 145 161 A discloses the use of the integrin inhibitor HM-3 for treating tumors.

According to the references, it is a common method to solve the problems of continuous administration due to short half-time via modifying or altering molecular structure. Chemical modification is the most widely used one. The chemical modifiers commonly used include polyethylene glycol (PEG), dextran, polyamino acids, polyanhydrides and so on. PEG has been approved as pharmaceutical auxiliary material and modifier by the U.S. Food and Drug Administration (FDA) because of the feature of non- toxicity, non-immunogenicity, and good Twater solubility. After PEG modification, he molecular weight of protein drugs increases while glomerular filtration rate decrease. PEG barrier protects protein from being hydrolyzed by protein proteolysis, meantime reduces the generation of neutralizing antibodies, which can extend the biological half-life of said protein drugs. Nowadays, there are various PEG modified protein drugs on sale. However, PEG modification can also affect the biological activity of protein, and the degree of influence relates to modifier, modifying conditions and the nature of the protein itself. For specific proteins, its best modification is determined by proteins modified by PEG and its biological activity. Although the study of the synthesis of small molecular polypeptide modified by PEG started recently, it has attracted a lot of attention from researchers.

**Contents of the invention**

**Objective of invention**

The invention made a further research for mPEG - SC - Ile-Val-Arg-Arg-Ala-Asp-Arg-Ala-Ala-Val-Pro-Gly-Gly-Gly- Gly-Arg-Gly-Asp, and discovered that it had therapeutic effect under the condition of decreasing the frequency of administration.

**Technical programs**

Disclosed herein is an integrin inhibitor modified by polyethylene glycol, said integrin inhibitor designated as HM-3 and having the sequence mPEG-Ile-Val-Arg-Arg-Ala-Asp-Arg-Ala-Ala-Val-Pro-Gly-Gly-Gly-Gly -Arg-Gly-Asp, wherein the range of molecular weight of said mPEG-SC is 500-20000.

Also disclosed is the PEG modified integrin inhibitor HM-3 as mentioned above, wherein the molecular weight of mPEG-SC (Monomethoxy-polyethylene glycol- succinimidyl carbonate) is 20000.

The present invention relates to the PEG modified integrin inhibitor HM-3 as mentioned above, for use in the treatment of a tumour originated from stomach, or head and neck. Also disclosed is an application of the PEG modified integrin inhibitor HM-3 for use in the treatment of a tumor originated from skin, thyroid, pancreas, lung, esophagus, breast, kidney, gall bladder, colon / rectum, ovary, uterus, cervix, prostate, bladder, testicular primary / secondary cancer or sarcoma.

**Beneficial effects**

1 In order to extend the half-life of Ile- Val-Arg-Arg-Ala- Asp-Arg-Ala-Ala-Val-Pro-Gly-Gly- Gly- Gly-Arg-Gly-Asp, we used PEG of various molecular weights to modify this polypeptide and discovered that mPEG-SC$_{20k}$-HM-3 had the role of extending half-time of HM-3 at the same time does no impact the activity in vivo and vitro. A product modified by polypeptide is a novel molecular, which usually produces different effects compared to the molecular without modification. This invention has implemented many in vivo and vitro studies regarding the therapeutic effect of mPEG-SC$_{20k}$-HM-3 on many tumors and discovered that mPEG-SC$_{20k}$-HM-3 played a positive role in inhibiting the growth of many tumors, and expanded its social and economic value.

2 According to the research, sequence of Ile-Val-Arg-Arg-Ala-Asp-Arg-Ala-Ala-Val-Pro had the function of inhibiting angiogenesis of tumors. The sequence of Arg - Gly - Asp (RGD) was an important integrin ligand and therefore the RGD-containing Gly-Gly-Gly-Gly-Arg-Gly-Asp could also specifically recognize integrin. The integrin antagonist polypeptide according to present invention is based on the binding of Gly-Gly-Gly-Gly-Arg-Gly-Asp sequence of RGD at the C terminal of Ile-Val-Arg-Arg-Ala-Asp-Arg-Ala-Ala-Val-Pro, which had the function of integrin affinity and binding ability. Meantime, polyethylene glycol modification was made at the N terminal of said integrin antagonist polypeptide, and eventually formed the sequence: mPEG-SC$_{20k}$-Ile-Val-Arg-Arg-Ala-Asp-Arg-Ala-Ala-Val-Pro-Gly-Gly-Gly-Gly-Arg-Gly-As p, which contained PEG and an 18-amino acid polypeptide. Such RGD containing sequence having the ability of integrin affinity and binding. Research demonstrated that the targets are integrin $\alpha v\beta 3$ and $\alpha 5\beta 1$, but the main target is $\alpha v\beta 3$. In addition, such sequence contained angiogenesis inhibition sequence and is able to inhibit the angiogenesis and eventually reach the effects of preventing growth and metastasis of tumors. PEG is a kind of macromolecule polymer with unique physical and chemical properties. It has good biocompatibility and is non-toxic, non-antigenic. After undergoing PEG modification, not only the main biological functions of protein or polypeptide drugs remain unchanged, but also the PEG modified protein or polypeptide drugs are given many excellent characteristicss: (1) Increase stability, extend plasma half-life; (2) Reduce immunogenicity and antigenicity; (3) Reduce toxicity; (4) Reduce the possibility of degradation by hydrolytic enzymes and the rate of renal clearance; (5) Improve the distribution and dynamics of drug in the body.

The target and anti-tumor activity of mPEG-SC$_{20k}$ polypeptide remain unchanged after modification, the half-time is extended, the clearance is reduced and the immunogenicity and antigenicity. In addition, the frequency of administration is reduced from previously administered once daily to once every 2-3 days.

Inventors got the result that the integrin has advantages of significant anti-tumor effects, less side effects, less dose and less cost through many experiments, which demonstrated that the PEG modified integrin antagonist according to the present invention is scientific, reasonable, feasible and effective and can become treatment medicine for curing human anti-tumor. It provides novel ideas and perspectives for future drug development, and expanded its social and economic value.

The half time of HM-3 polypeptide before modification is 0.46h, and after modification by the mPEG-SC20k, the half time is 20.13h, as indicated in the table below.

**Table 1 The comparison of half-time between mPEG-SC$_{20k}$-HM-3 and HM-3 ($t_{1/2}\beta$ is half-time)**

| Medicine | $t_{1/2}\beta$ (h) | CL (L/h/kg) | AUC$_{0-\infty}$ (mg/L/h) | MRT$_{0-\infty}$ (h) |
|---|---|---|---|---|
| mPEG-SC$_{20k}$-HM-3 | 20.13 ±0.64 | 0.0071 ±0.0012 | 4391.22 ±3562.89 | 15.35 ±1.07 |
| HM-3 | 0.46 ±0.12 | 1.38 ±0.15 | 25.63 ±9.76 | 0.036 ±0.002 |

**Description of the Figures**

Figures 1, 2, 4 and 6 relate to Examples according to the invention. Figures 3, 5, 7 to 21 relate to Reference Examples.

**Figure 1:** The integrin antagonist polypeptide binded target via flow cytometry experiments. (a) showed the first test, and (b) was repeated test.

**Figure 2:** The comparison of the integrin antagonist polypeptide with HM-3 immunogenicity

**Figure 3:** The effect of tumor growth inhibition induced by integrin antagonist polypeptide on human esophageal cancer Ec109 xenograft tumors in nude mice

**Figure 4:** The effect of tumor growth inhibition induced by integrin antagonist polypeptide on human nasopharyngeal cancer CNE xenograft tumors in nude mice

**Figure 5:** The effect of tumor growth inhibition induced by integrin antagonist polypeptide on human Thyroid cancer SW-579 xenograft tumors in nude mice

**Figure 6:** The effect of tumor growth inhibition induced by integrin antagonist polypeptide on human gastric cancer MGC803 xenograft tumors in nude mice

**Figure 7:** The effect of tumor growth inhibition induced by integrin antagonist polypeptide on human pancreatic cancer SW-1990 xenograft tumors in nude mice

**Figure 8:** The effect of tumor growth inhibition induced by integrin antagonist polypeptide on human lung cancer H460 xenograft tumors in nude mice

**Figure 9:** The effect of tumor growth inhibition induced by integrin antagonist polypeptide on human breast cancer MDA-MB-231 xenograft tumors in nude mice

**Figure 10:** The effect of tumor growth inhibition induced by integrin antagonist polypeptide on human gallbladder cancer GBC-SD xenograft tumors in nude mice

**Figure 11:** The effect of tumor growth inhibition induced by integrin antagonist polypeptide on human kidney cancer A498 xenograft tumors in nude mice

**Figure 12:** The effect of tumor growth inhibition induced by integrin antagonist polypeptide on human colon cancer HT-29 xenograft tumors in nude mice

**Figure 13:** The effect of tumor growth inhibition induced by integrin antagonist polypeptide on human ovarian cancer SK-OV-3 xenograft tumors in nude mice

**Figure 14:** The effect of tumor growth inhibition induced by integrin antagonist polypeptide on human endometrial cancer HHUA xenograft tumors in nude mice

**Figure 15:** The effect of tumor growth inhibition induced by integrin antagonist polypeptide on human cervical cancer HeLa xenograft tumors in nude mice

**Figure 16:** The tumor picture of human cervical cancer HeLa xenograft tumors in nude mice inhibited by integrin antagonist polypeptide

**Figure 17:** The effect of tumor growth inhibition induced by integrin antagonist polypeptide on human prostate cancer DU-145 xenograft tumors in nude mice

**Figure 18:** The effect of tumor growth inhibition induced by integrin antagonist polypeptide on human bladder cancer HT1376 xenograft tumors in nude mice

**Figure 19:** The tumor picture of human bladder cancer HT1376 xenograft tumors in nude mice inhibited by integrin antagonist polypeptide

**Figure 20:** The effect of tumor growth inhibition induced by integrin antagonist polypeptide on human testicular cancer 5637 xenograft tumors in nude mice

**Figure 21:** The effect of tumor growth inhibition induced by integrin antagonist polypeptide on sarcoma HT-1080 xenograft tumors in nude mice

## Detail embodiments

Examples 1 to 6, 8 and 10 are Examples according to the invention. Examples 7, 9, 11 to 25 are Reference Examples.

## Example 1 Integrin antagonist HM-3

The method of synthesizing solid-state of Ile-Val-Arg-Arg-Ala-Asp-Arg-Ala-Ala-Val-Pro-Gly-Gly-Gly-Gly-Arg-Gly-Asp: Fmoc-Ile-wang resin or Fmoc-Ile-CTC resin is regarded as a raw material and then use protected amino acids sequentially connect from Dipeptides to Octadecapeptide , then washed thoroughly, followed by peptide cut and post-treatment to obtain a crude product of HM-3. The crude product was purified. At first, be dissolved, and then purified twice by preparative HPLC, and finally lyophilized to acquire pure product. Specific steps are as follows:

### 1. Synthesis

Weigh the Fmoc-Ile-Wang resin14.7g, and then pour it into a glass sand core reaction column of 1L volume. The CH2Cl2 147ml was added to make resin full expansion.

Uncapping: After the solution of uncapping 25ml with hexahydropyridine / DMF is sealed, keep it in the shaker for 5 minutes in room temperature. Then the uncapping solution is drained and washed once again with DMF in the middle and then add 20% uncapping solution 25ml with a reaction of 15 minutes;

Washing: Drained the uncapping solution and then washed the resin six times with the DMF, drained, and then take 20 resin into a small test tube, add test reagent, and heated under the tempreture of 115 ° C for 3 minutes.

Condensating: Weigh protective amino acids and HOBt 2.025g, then be dissolved in 15ml DMF and 2.33ml DIC, then pour it into the reactor lasting about 1.5 hours, the temperature is controlled at about 34 ° C.

Washing: Drain the reaction solution, and wash the resin with DMF three times, drain again, and then put 10-20 resin particles into a small test tube, add test toner and heat it at 115 ° C 3-5 min.

Cutting: Put the drained resin into round bottom flask of 500mL volume, and then add 90% cleavage solution of 300mL (phenol: thioanisole: EDT = 90: 3:3:2:2), and seal for 2 hours .

Separate polypeptide from resin with a frit funnel.

Postconditioning: At first add anhydrous ether to cleavage solution to precipitate the polypeptide, then centrifuge, then the supernatant was discarded, and then polypeptide is washed with anhydrous ether six times. Finally, drain it to gain crude peptide 9.5g

### 2. Purification

Dissolving: Weigh ID-18 crude product exactly, then add purified water to the appropriate configuration of 10g / 1 solution. Ultrasonic stirs until a clear solution without particulate comes up.

Filtrating: The ID-18 solution is filtrated with 0.45um membrane of sand core filter Producing:

The primary purification

Balancing: Configure the solution of 5% acetonitrile and 95% aqueous trifluoroacetic acid. Rinse 10 min at a flow rate 80ml/min.

Loading: Load with the infusion pump, flow rate 80ml/min. And collect baseline and solution which is greater in the UV wavelength absorption of 220nm than 200mv and detect whether there are samples out.

Eluting: Gradient of elution

| Time (min) | Velocity of flow (ml / min) | Acetonitrile % | Aqueous trifluoroacetic acid% |
|---|---|---|---|
| 0 | 80 | 8 | 92 |
| 40 | 80 | 18 | 82 |

Collect solution which is stronger in the UV wavelength absorption of 220nm than 200mvUV. The purity of greater than 95% is regarded as a peak and ready to do a secondary separation and purification.

The secondary purification

Balancing: Configure the solution of 5% acetonitrile +95% aqueous acetic acid. Rinse 10 min at a flow rate 80ml/min.

Loading: Load the primary peak with infusion pump after evaporating the organic solvent. And collect baseline and solution which is greater in the UV wavelength absorption of 220nm than 200mv to detect whether there are samples out.

Eluting: Gradient of elution

| Time (min) | Velocity of flow (ml / min) | Acetonitrile % | aqueous acetic acid % |
|---|---|---|---|
| 0 | 80 | 5 | 95 |
| 40 | 80 | 15 | 85 |

Collect the solution which is stronger in the UV wavelength absorption of 220nm than 200mv. The purity of greater than 99% is regarded as qualification. Concentrating, filtering and freeze-drying: Concentrate the qualified solution with a rotary evaporator under 37 ° C and remove residual solvent and water. Finally filter it with 0.22um filter membrane and put the filtrate into the freeze-dried pan, then freeze and dry it with a freeze dryer to give pure product.

**Example 2 Steps of polyethylene glycol modifying polypeptide**

The reaction of mPEG-SC20K and the HM-3

Weigh 2g mPEG-SC20k and 106.24 mg HM-3 (molar ratio 1.5:1) respectively. Both of them are placed in 40ml-100ml pH 5-8.5 PBS buffer solution at the conditions at 4 ° C overnight and allow them to react. PEG-SC500-20000 can be connected as described in Example 2 to produce modified polypeptides

**Example 3 Steps of separation and purification**

**1. Separation**

The sample after the reaction is purified through semi-preparative HPLC (HPLC, BIO-RAD) and purification conditions are as follows:

Mobile phase: ACN (+0.1% TFA), $H_2O$ (+0.1% TFA); ACN linear gradient: 40% -95%;

Flow rate: 2 ml / min; Running time: 12 min;

Loading volume: 1.0 ml; detecting wave length: 220 nm.

Semi-preparative column: YMC, 250 mm 10 mm (5 μ m packing).

In the process of peaks of the peak, the product was collected by centrifugation tube

**2. Purification**

The collected products through HPLC are frozen in the cryogenic freezer at -70 ° C overnight, then freeze and dry them through the freeze dryer until the products become white powder (30 h or so). Gain lyophilized product, weigh and record the weight of the products, and then save them in the -20 ° C refrigerator and make identification.

1 Analysis of purity of the products

The products are lyophilized and analyzed by analytical HPLC. The conditions of purity analysis are as follows: Mobile phase: ACN (+0.1% TFA), H2O (+0.1% TFA); ACN linear gradient: 10% -100%;
Flow rate: 1 ml / min;
Running time: 15 min;
Sample volume: 20 μl; detection wavelength: 220 nm.
Analytical Column: Beijing innovation Tongheng, 250 mm x4.6 mm (5μm filler)
2 SDS-PAGE analysis of modified products

Basic operation reference "Molecular Cloning (The second edition)." Concentration of stacking gel is 5%, and separating gel 10%, concentrated voltage 80 volts, separation voltage 120 volts. The bands of samples are stained with Bal2 first after electrophoresis especially for the portion containing PEG;. Coomassie brilliant blue R250 as a Marker stains part of the protein. Place it into bleaching solution after staining to the effect of background transparent and then analyze the results through scanning. The following takes mPEG20000 modified polypeptide integrin antagonist polypeptide (mPEG-SC20k-HM-3) for example to make description. Integrin antagonist polypeptide described in example is the integrin antagonist polypeptide modified by mPEG20000.

**Example 4 Study of pharmacokinetics of mPEG-SC20k-HM-3 in rats**

SD rats were randomly divided into six groups with the same number for male and female. Take three groups were adminstrated intravenously integrin antagonist polypeptide with a high dose of 52 mg / kg (equivalent to HM-3 4.2 mg / kg), an intermediate dose of 26 mg / kg (equivalent to HM-3 2.1mg/kg), a low-dose of 13 mg / kg (equivalent to HM-3 1.05 mg / kg). The other 3 groups were injected HM-3 with a high dose of 4.2 mg / kg, an intermediate dose of 2.1mg/kg, a low doses of 1.05 mg / kg. Collected whole blood 0.5 ml once from the orbital venous plexus after 0.5h, 1h, 2h, 3h, 6h, 12h, 24h, 48h, 72h, 96h, 108h, 132h of drug administration, and applied heparin to get the effect of anti-coagulation. 12000 rpm/2min centrifuged plasma. Draw supernatant 200 μl and 80 ° C preheated PBS (0.05M pH7.4) buffer 600 μ 1 and mixed. Bath in the 80 ° C water for 30 min. Centrifuged 2 min at 12000 rpm, and collected the supernatant, and stored in the condition of -20 ° C. Dissolved at room temperature, measured integrin antagonist peptide plasma concentration by ELISA.

**The comparison of pharmacokinetic parameters of mPEG-SC20k-HM-3 and HM-3 in SD rats ($t_{1/2}\beta$ halt-time, CL the rate of plasma clearance, AUD Area under the curve, MRT the average residence time**

| Drugs | $t_{1/2}p$ (min) | CL (L/h/kg) | $AUC_{0-\infty}$ (mg/L/h) | $MRT_{0-\infty}$ (min) |
|---|---|---|---|---|
| mPEG-SC$_{20k}$-HM-3 | 1207.80±38.40 | 0.0071±0.0012 | 4391.22±3562.89 | 921.18±64.29 |
| HM-3 | 27.66±7.37 | 1.38±0.15 | 25.63±9.76 | 2.17±0.13 |

As known from Table 1, compared with the HM-3, mPEG-SC$_{20k}$-HM-3 has a significantly longer half-life and lower plasma clearance. The above experimental data have demonstrated PEG-modified protein can significantly improve the feature of pharmacokinetics of polypeptide drug in rats

**Example 5 Analyzing the combination of mPEG-SC$_{2ok}$-HM-3 and targets through flow cytometry**

(1) Bel-7402 tumor cells and MCF-7 fusion cultured to 80% after the 24-well plate, trypsinized collected, washed with ice-cold PBS twice, the cells were labeled prior containing 1% BSA resuspended in PBS 30 min.

(2) 2 μl mouse anti-human αvβ3 function-blocking monoclonal antibody (1.0 μg/μl,1:200), mouse anti-human 2 μl α5β1 function-blocking monoclonal antibody (1.0 μg/μl,, 1:200) and the cell suspension were incubated at 4 ° C 1.5h.

(3) collected the labeled cells and washed with ice-cold PBS twice, followed by 100μl FITC labeled polypeptide modified mPEG-SC$_{20k}$-HM-3 (2 mg / ml) and the cell suspension were incubated at 4 ° C incubate 1.5h

(4) After labeled, the cells were collected and washed with ice-cold PBS 2 times, followed by resuspension with 400μl PBS and analyzed by flow cytometry, and detected intensity of the FITC fluorescence through channel FL1.

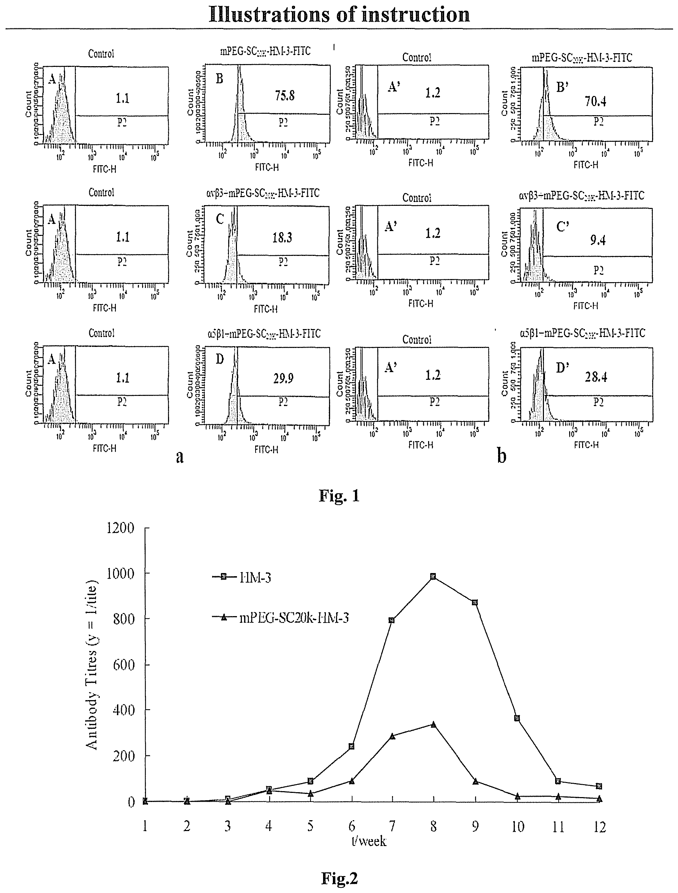

Figure 1 showed mPEG-SC20k-HM-3 was able to bind $\alpha$v$\beta$3 and $\alpha$5$\beta$1 of integrin, but the main target was still $\alpha$v$\beta$3. It demonstrated that the main targets of HM-3 were not changed after PEG modifying, and the active sites are not covered by PEG.

**Example 6 The assay of immunogenicity of mPEG-SC$_{20k}$-HM-3 and HM-3**

BALB/c white mice were randomly divided into 2 groups, and the same number of male and female. 36 mg/kg mPEG-SC$_{20k}$-HM-3 and 3.0 mg/kg HM-3 were administrated intravenously through tail vein, respectively, lasting 8 weeks, and collected blood from the orbital venous plexus once a week during 1-12 weeks. Centrifuged 2 min at 12000 rpm, collected the supernatant, and stored in the condition of-20 ° C. Dissolved at room temperature, 0.1 ml was used to measure antibody titers in serum by ELISA.

**Group settings:**

Group 1 The effective dose of HM-3 was 3.0 mg/kg. Administration: once a day, 6 rats (male,3; female, 3)

Group 2 The effective dose of mPEG-SC$_{20k}$-HM-3 was 36 mg/kg. Administration: once two days, 6 rats (male,3; female, 3)

Figure 2 showed that antibody appeared at the third week of administration in HM-3 group, whereas lower titer antibody was detected at the fifth week of administration in mPEG-SC$_{20k}$-HM-3 group. The antibody titer of integrin antagonist polypeptide group was significantly lower than HM-3 group at any time points. After cession of administration, antibody titer decreased gradually, and the antibody in integrin antagonist polypeptide group could not detect at the twelfth week of administration, which demonstrated that PEG modification could significantly reduce the immunogenicity of HM-3 in vivo.

**Example 7 The experiment of tumor growth inhibition induced by mPEG-SC$_{20k}$-HM-3 on human esophageal cancer Ec109 xenograft tumors in nude mice**

Picked the tumor tissue of vigorous growth and was cut into pieces of about 1.5mm$^3$. Under sterile conditions, the tumor tissue was inoculated subcutaneously on the right of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein, once 3 days. The mice in the placebo group were injected saline with the same volume. Tumor volume formula:

$$TV = 0.52 \times a \times b^2$$

a, b respectively represent the length and width. According to the result of measurement, calculate the relative tumor volume. Evaluation of antitumor activity is relative tumor proliferation rate T / C (%). The formula is as follows:

$$T/C(\%) = T_{RTV} / C_{RTV} \times 100\%$$

$T_{RTV}$: Treatment group RTV; $C_{RTV}$: negative control group RTV

**Table 2 The experiment of tumor growth inhibition induced by mPEG-SC$_{20k}$-HM-3 on human esophageal cancer Ec109 xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once 1 day | 21.43 | 12 | 21.22 | 12 | 0.926 | - |
| Taxol | 10 | Once 2 days | 20.56 | 8 | 20.24 | 8 | 0.276 | 70.19% |
| Endostar | 2.5 | Once 1 day | 21.55 | 8 | 21.26 | 8 | 0.602 | 35.0% |
| HM-3 | 1.5 | Once 3 days | 21.42 | 8 | 20.74 | 8 | 0.365 | 60.57% |
| mPEG-SC$_{20K}$-HM-3 high | 36.7 | Once 3 days | 22.20 | 8 | 21.75 | 8 | 0.183 | 62.24%* |
| mPEG-SC$_{20k}$-HM-3 intermediate | 18.75 | Once 3 days | 20.45 | 8 | 19.93 | 8 | 0.318 | 55.66% |
| mPEG-SC$_{20k}$-HM-3 low | 9.38 | Once 3 days | 21.22 | 8 | 20.38 | 8 | 0.406 | 50.16% |

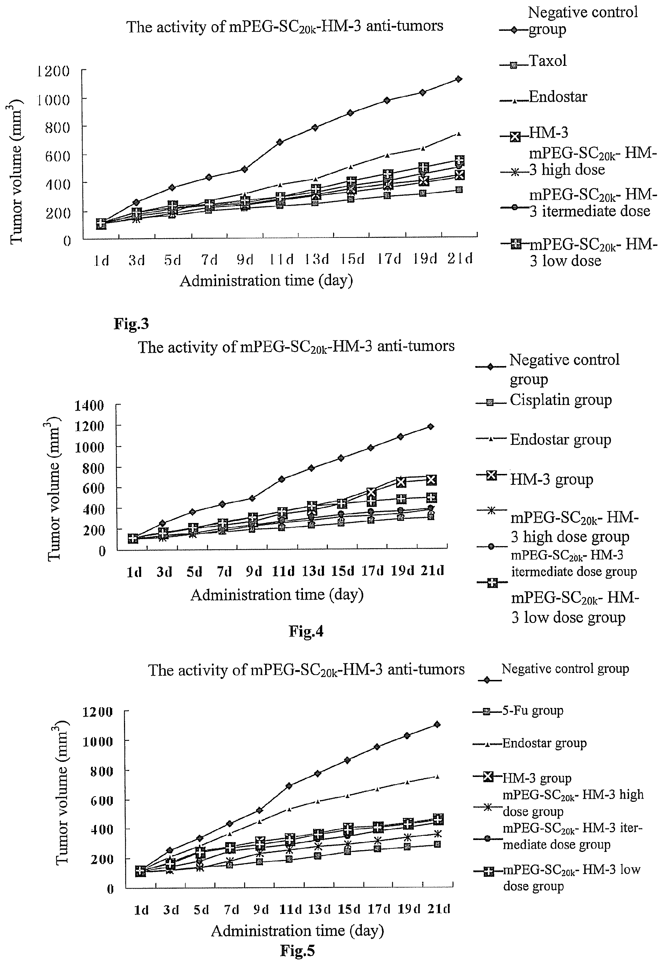

Results: shown in table 2 and figure 3. Taxol group: 10mg/kg, the rate of inhibiting tumor of nude mice with human esophageal cancer Ec109 is 70.19%; Endostar group: 2.5mg/kg, the rate of inhibiting tumor of nude mice with human esophageal Ec109 is 35.0%; HM-3 group: 1.5mg/kg, the rate of inhibiting tumor of nude mice with human esophageal Ec109 is 60.57%; The rates of inhibiting tumor of nude mice with human esophageal Ec109 in groups of high, intermediate and low dose of polypeptide are 62.24%, 55.66%, 50.16% respectively.

The results of experiment of tumor growth inhibition induced by mPEG-SC$_{20k}$-HM-3 on human esophageal cancer Ec109 xenograft tumors in nude mice demonstrated that compared with negative control group, mPEG-SC$_{20K}$-HM-3 36.7mg/kg played a significant inhibition role in growth of human esophageal cancer Ec109. *$P<0.05$(there is significant difference compared with negative group)

**Example 8 The experiment of tumor growth inhibition induced by mPEG-SC$_{20k}$-HM-3 on human nasopharyngeal cancer CNE xenograft tumors in nude mice**

Picked the tumor tissue of vigorous growth and was cut into pieces of about 1.5mm$^3$. Under sterile conditions, the tumor tissue was inoculated subcutaneously on the right of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein, once 3 days. The mice in the placebo group were injected saline with the same volume. Tumor volume formula:

$$TV=0.52 \times a \times b^2$$

a, b respectively represent the length and width. According to the result of measurement, calculate the relative tumor volume. Evaluation of antitumor activity is relative tumor proliferation rate T / C (%). The formula is as follows:

T/C(%)=$T_{RTV}$/ $C_{RTV}$ $\times$ 100%

$T_{RTV}$: Treatment group RTV; $C_{RTV}$: negative control group RTV

**Table 3 The effect of tumor growth inhibition induced by mPEG-SC$_{20k}$-HM-3 on human nasopharyngeal cancer CNE xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once 1 day | 22.78 | 12 | 22.37 | 12 | 1.201 | - |
| Cisplatin | 10 | Twice 1 week | 22.35 | 8 | 22.33 | 7 | 0.320 | 73.36% |
| Endostar | 2.5 | Once 1 day | 22.43 | 8 | 22.39 | 8 | 0.757 | 37.01% |
| HM-3 | 1.5 | Once 3 days | 22.60 | 8 | 22.85 | 8 | 0.468 | 61.03% |
| mPEG-SC$_{20k}$-HM-3 high | 36.7 | Once 3 days | 22.16 | 8 | 22.15 | 8 | 0.382 | 68.22%* |
| mPEG-SC$_{20k}$-HM-3 intermediate | 18.75 | Once 3 days | 22.75 | 8 | 24.00 | 8 | 0.406 | 66.19% |
| mPEG-SC$_{20k}$-HM-3 low | 9.38 | Once 3 days | 23.02 | 8 | 23.70 | 8 | 0.537 | 55.32% |

Results: shown in table 3 and figure 4. Cisplatin group: 10mg/kg, the rate of inhibiting tumor of nude mice with human nasopharyngeal cancer CNE is 73.36%, but significantly affects the weight of experimental animals; Endostar group: 2.5mg/kg, the rate of inhibiting tumor of nude mice with human nasopharyngeal cancer CNE is 37.01%; HM-3 group: 1.5mg/kg, the rate of inhibiting tumor of nude mice with human nasopharyngeal cancer CNE is 61.03%; The rates of inhibiting tumor of nude mice with human nasopharyngeal cancer CNE in groups of high, intermediate and low dose of polypeptide are 68.22%, 66.19%, 55.32% respectively, but there is no significant influence on the weight of experimental mice.

The results of experiment of tumor growth inhibition induced by mPEG-SC$_{20k}$-HM-3 on human nasopharyngeal cancer CNE xenograft tumors in nude mice demonstrated that compared with negative control group, mPEG-SC$_{20K}$-HM-3 36.7mg/kg played a significant inhibition role in growth of human nasopharyngeal cancer CNE; compared with positive cisplatin group, there was no significant influence on the weight of experimental mice, and no obvious toxic and side effects. *$P<0.05$(there is significant difference compared with negative group)

**Example 9 The experiment of tumor growth inhibition induced by mPEG-SC$_{20k}$-HM-3 on human thyroid cancer SW-579 xenograft tumors in nude mice**

Picked the tumor tissue of vigorous growth and was cut into pieces of about 1.5mm$^3$. Under sterile conditions, the tumor tissue was inoculated subcutaneously on the right of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein, once 3 days. The mice in the placebo group were injected saline with the same volume. Tumor volume formula:

$$TV = 0.52 \times a \times b^2$$

a, b respectively represent the length and width. According to the result of measurement, calculate the relative tumor volume. Evaluation of antitumor activity is relative tumor proliferation rate T / C (%). The formula is as follows:
T/C(%)=$T_{RTV}$/ $C_{RTV}$ $\times$ 100%

$T_{RTV}$: Treatment group RTV; $C_{RTV}$: negative control group RTV

**Table 4 The effect of tumor growth inhibition induced by mPEG-SC$_{20k}$-HM-3 on human thyroid cancer SW-579 xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once 1 day | 21.34 | 12 | 21.23 | 12 | 1.253 | - |
| 5-Fu | 10 | Once 1 day | 21.56 | 8 | 21.32 | 7 | 0.320 | 74.50% |
| Endostar | 2.5 | Once 1 day | 21.67 | 8 | 20.39 | 8 | 0.850 | 32.20% |
| HM-3 | 1.5 | Once 1 day | 22.76 | 8 | 22.70 | 8 | 0.530 | 57.70% |
| mPEG-SC$_{20k}$-HM-3 high | 36.7 | Once 3 day | 22.32 | 8 | 21.76 | 8 | 0.406 | 67.63%* |
| mPEG-SC$_{20k}$-HM-3 intermediate | 18.75 | Once 3 day | 22.90 | 8 | 22.42 | 8 | 0.494 | 60.56% |
| mPEG-SC$_{20k}$-HM-3 low | 9.38 | Once 3 day | 21.58 | 8 | 21.28 | 8 | 0.521 | 58.42% |

Results: shown in table 4 and figure 5. 5-Fu group: 10mg/kg, the rate of inhibiting tumor of nude mice with human thyroid cancer SW-579 is 74.50%, but 5-Fu has greater toxicity and leads to the weight loss and death of experimental animals; Endostar group: 2.5mg/kg, the rate of inhibiting tumor of nude mice with human thyroid cancer SW-579 is 32.20%; HM-3 group: 1.5mg/kg, the rate of inhibiting tumor of nude mice with human thyroid cancer SW-579 is 57.70%; The rates of inhibiting tumor of nude mice with human thyroid cancer SW-579 in groups of high, intermediate and low dose of polypeptide are 67.63%, 60.56%, 58.42%, respectively, but there is no significant influence on the weight of experimental mice.

The results of experiment of tumor growth inhibition induced by mPEG-SC$_{20k}$-HM-3 on human thyroid cancer SW-579 xenograft tumors in nude mice demonstrated that compared with negative control group, mPEG-SC$_{20K}$-HM-3 36.7mg/kg played a significant inhibition role in growth of human thyroid cancer SW-579; compared with positive 5-Fu group, there was no significant influence on the weight of experimental mice, and no obvious toxic and side effects.

*$P<0.05$(there is significant difference compared with negative group).

**Example 10 The experiment of tumor growth inhibition induced by mPEG-SC$_{20k}$-HM-3 on human gastric cancer MGC803 xenograft tumors in nude mice**

Picked the tumor tissue of vigorous growth and was cut into pieces of about 1.5mm$^3$. Under sterile conditions, the tumor tissue was inoculated subcutaneously on the right of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein, once 3 days. The mice in the placebo group were injected saline with the same volume. Tumor volume formula:

$$TV = 0.52 \times a \times b^2$$

a, b respectively represent the length and width. According to the result of measurement, calculate the relative tumor volume. Evaluation of antitumor activity is relative tumor proliferation rate T / C (%). The formula is as follows:
$T/C(\%) = T_{RTV} / C_{RTV} \times 100\%$

$T_{RTV}$: Treatment group RTV; $C_{RTV}$: negative control group RTV

**Table 5 The effect of tumor growth inhibition induced by mPEG-SC$_{20k}$-HM-3 on human gastric cancer MGC803 xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once 1 day | 22.32 | 12 | 22.15 | 12 | 0.723 | -- |
| Taxol | 10 | Once 2 days | 22.14 | 8 | 22.05 | 8 | 0.187 | 74.12% |
| Endostar | 2.5 | Once 1 day | 23.22 | 8 | 23.13 | 8 | 0.504 | 30.29% |
| HM-3 | 1.5 | Once 3 days | 22.52 | 8 | 22.37 | 8 | 0.214 | 70.40% |
| mPEG-SC$_{20k}$-HM-3 high | 36.7 | Once 3 days | 22.67 | 8 | 22.24 | 8 | 0.192 | 73.42%* |
| mPEG-SC$_{20k}$-HM-3 intermediate | 18.75 | Once 3 days | 22.76 | 8 | 22.32 | 8 | 0.218 | 69.86% |
| mPEG-SC$_{20k}$-HM-3 low | 9.38 | Once 3 days | 22.81 | 8 | 22.44 | 8 | 0.292 | 59.57% |

Results: shown in table 4 and figure 5. Taxol group: 10mg/kg, the rate of inhibiting tumor of nude mice with human gastric cancer MGC803 is 74.12%; Endostar group: 2.5mg/kg, the rate of inhibiting tumor of nude mice with human gastric cancer MGC803 is 32.29%; HM-3 group: 1.5mg/kg, the rate of inhibiting tumor of nude mice with human gastric cancer MGC803 is 70.40%; The rates of inhibiting tumor of nude mice with human gastric cancer MGC803 in groups of high, intermediate and low dose of polypeptide are 73.42%, 69.86%, 59.57%, respectively.

The results of experiment of tumor growth inhibition induced by mPEG-SC$_{20k}$-HM-3 on human gastric cancer MGC803 xenograft tumors in nude mice demonstrated that compared with negative control group, mPEG-SC$_{20K}$-HM-3 36.7mg/kg played a significant inhibition role in growth of human gastric cancer MGC803. *P<0.05* (there is significant difference compared with negative group).

**Example 11 The experiment of tumor growth inhibition induced by mPEG-SC$_{20k}$-HM-3 on human pancreatic cancer SW-1990 xenograft tumors in nude mice**

Picked the tumor tissue of vigorous growth and was cut into pieces of about 1.5mm$^3$. Under sterile conditions, the tumor tissue was inoculated subcutaneously on the right of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein, once 3 days. The mice in the placebo group were injected saline with the same volume. Tumor volume formula:

$$TV = 0.52 \times a \times b^2$$

a, b respectively represent the length and width. According to the result of measurement, calculate the relative tumor volume. Evaluation of antitumor activity is relative tumor proliferation rate T / C (%). The formula is as follows:

$$T/C(\%) = T_{RTV} / C_{RTV} \times 100\%$$

$T_{RTV}$: Treatment group RTV; $C_{RTV}$: negative control group RTV

**Table 6 The effect of tumor growth inhibition induced by mPEG-SC$_{20k}$-HM-3 on human pancreatic cancer SW-1990 xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once 1 day | 22.30 | 12 | 22.18 | 12 | 1.385 | - |
| 5-Fu | 10 | Once 1 day | 23.17 | 8 | 22.43 | 8 | 0.323 | 76.68% |
| Endostar | 2.5 | Once 1 day | 24.26 | 8 | 23.73 | 8 | 0.932 | 32.71 % |
| HM-3 | 1.5 | Once 3 days | 23.40 | 8 | 23.10 | 8 | 0.582 | 57.94% |
| mPEG-SC$_{20k}$-HM-3 hight | 36.7 | Once 3 days | 24.63 | 8 | 23.32 | 8 | 0.446 | 67.76%* |
| mPEG-SC$_{20k}$-HM-3 intermediate | 18.75 | Once 3 days | 23.33 | 8 | 23.17 | 8 | 0.491 | 64.55% |
| mPEG-SC$_{20k}$-HM-3 low | 9.38 | Once 3 days | 23.34 | 8 | 22.67 | 8 | 0.687 | 50.40% |

Results: shown in table 4 and figure 5. 5-Fu group: 10mg/kg, the rate of inhibiting tumor of nude mice with human pancreatic cancer SW-1990 is 76.68%; Endostar group: 2.5mg/kg, the rate of inhibiting tumor of nude mice with human pancreatic cancer SW-1990 is 32.71%; HM-3 group: 1.5mg/kg, the rate of inhibiting tumor of nude mice with human pancreatic cancer SW-1990 is 57.94%; The rates of inhibiting tumor of nude mice with human pancreatic cancer SW-1990 in groups of high, intermediate and low dose of polypeptide are 67.76%, 64.55%, 50.40%, respectively.

The results of experiment of tumor growth inhibition induced by mPEG-SC$_{20k}$-HM-3 on human pancreatic cancer SW-1990 xenograft tumors in nude mice demonstrated that compared with negative control group, mPEG-SC$_{20K}$-HM-3 36.7mg/kg played a significant inhibition role in growth of human pancreatic cancer SW-1990. *P<0.05*(there is significant difference compared with negative group).

**Example 12 The experiment of tumor growth inhibition induced by mPEG-SC$_{20k}$-HM-3 on human lung cancer H460 xenograft tumors in nude mice**

Picked the tumor tissue of vigorous growth and was cut into pieces of about 1.5mm$^3$. Under sterile conditions, the tumor tissue was inoculated subcutaneously on the right of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein, once 3 days. The mice in the placebo group were injected saline with the same volume. Tumor volume formula:

$$TV=0.52×a×b^2$$

a, b respectively represent the length and width. According to the result of measurement, calculate the relative tumor volume. Evaluation of antitumor activity is relative tumor proliferation rate T / C (%). The formula is as follows:

$T/C(\%)=T_{RTV}/ C_{RTV} \times 100\%$

$T_{RTV}$: Treatment group RTV; $C_{RTV}$: negative control group RTV

**Table 7 The effect of tumor growth inhibition induced by mPEG-SC$_{20k}$-HM-3 on human lung cancer H460 xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once 1 day | 21.36 | 12 | 21.21 | 12 | 0.794 | - |
| Taxol | 10 | Once 2 days | 22.34 | 8 | 19.58 | 8 | 0.248 | 68.77% |
| Endostar | 2.5 | Once 1 day | 21.08 | 8 | 20.86 | 8 | 0.546 | 31.20% |
| HM-3 | 1.5 | Once 3 days | 21.33 | 8 | 21.11 | 8 | 0.274 | 65.42% |
| mPEG-SC$_{20k}$-HM-3high | 36.7 | Once 3 days | 21.47 | 8 | 20.79 | 8 | 0.266 | 66.45%* |
| mPEG-SC$_{20k}$-HM-3intermediate | 18.75 | Once 3 days | 22.38 | 8 | 22.16 | 8 | 0.354 | 55.37% |
| mPEG-SC$_{20k}$-HM-3low | 9.38 | Once 3 days | 21.28 | 8 | 21.06 | 8 | 0.363 | 54.28% |

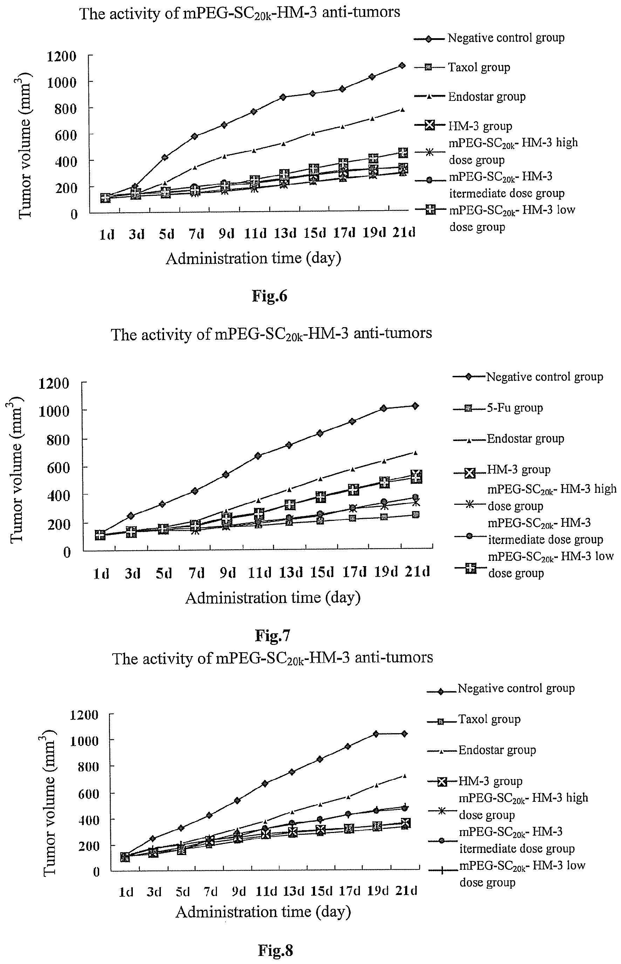

Results: shown in table 7 and figure 8. Taxol group: 10mg/kg, the rate of inhibiting tumor of nude mice with human lung cancer H460 is 68.77%; Endostar group: 2.5mg/kg, the rate of inhibiting tumor of nude mice with human lung cancer H460 is 31.20%; HM-3 group: 1.5mg/kg, the rate of inhibiting tumor of nude mice with human lung cancer H460 is 65.42%; The rates of inhibiting tumor of nude mice with human lung cancer H460 in groups of high, intermediate and low dose of polypeptide are 66.45%, 55.37%, 54.28%, respectively.

The results of experiment of tumor growth inhibition induced by mPEG-SC$_{20k}$-HM-3 on human lung cancer H460 xenograft tumors in nude mice demonstrated that compared with negative control group, mPEG-SC$_{20K}$-HM-3 36.7mg/kg played a significant inhibition role in growth of human lung cancer H460. *$P<0.05$(there is significant difference compared with negative group).

**Example 13 The experiment of tumor growth inhibition induced by mPEG-SC$_{20k}$-HM-3 on human breast cancer MDA-MB-231 xenograft tumors in nude mice**

Picked the tumor tissue of vigorous growth and was cut into pieces of about 1.5mm$^3$. Under sterile conditions, the tumor tissue was inoculated subcutaneously on the right of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein, once 3 days. The mice in the placebo group were injected saline with the same volume. Tumor volume formula:

$$TV=0.52 \times a \times b^2$$

a, b respectively represent the length and width. According to the result of measurement, calculate the relative tumor volume. Evaluation of antitumor activity is relative tumor proliferation rate T / C (%). The formula is as follows:

$T/C(\%){=}T_{RTV}/ C_{RTV} \times 100\%$

$T_{RTV}$: Treatment group RTV; $C_{RTV}$: negative control group RTV

**Table 8 The effect of tumor growth inhibition induced by mPEG-SC$_{20k}$-HM-3 on human breast cancer MDA-MB-231 xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | -- | Once 1 day | 22.32 | 10 | 22.69 | 10 | 1.243 | -- |
| Taxol | 10 | Once 2 days | 23.90 | 8 | 23.32 | 8 | 0.335 | 73.05% |
| Endostar | 2.5 | Once 1 day | 24.65 | 8 | 23.03 | 8 | 0.801 | 35.57% |
| HM-3 | 1.5 | Once 3 days | 23.43 | 8 | 23.54 | 8 | 0.533 | 57.14% |
| mPEG-SC$_{20k}$-HM-3high | 36.7 | Once 3 days | 22.38 | 8 | 22.73 | 8 | 0.459 | 63.05%* |
| mPEG-SC$_{20k}$-HM-3intermediate | 18.75 | Once 3 days | 23.66 | 8 | 22.34 | 8 | 0.508 | 59.11% |
| mPEG-SC$_{20k}$-HM-3low | 9.38 | Once 3 days | 23.73 | 8 | 23.49 | 8 | 0.609 | 51.01% |

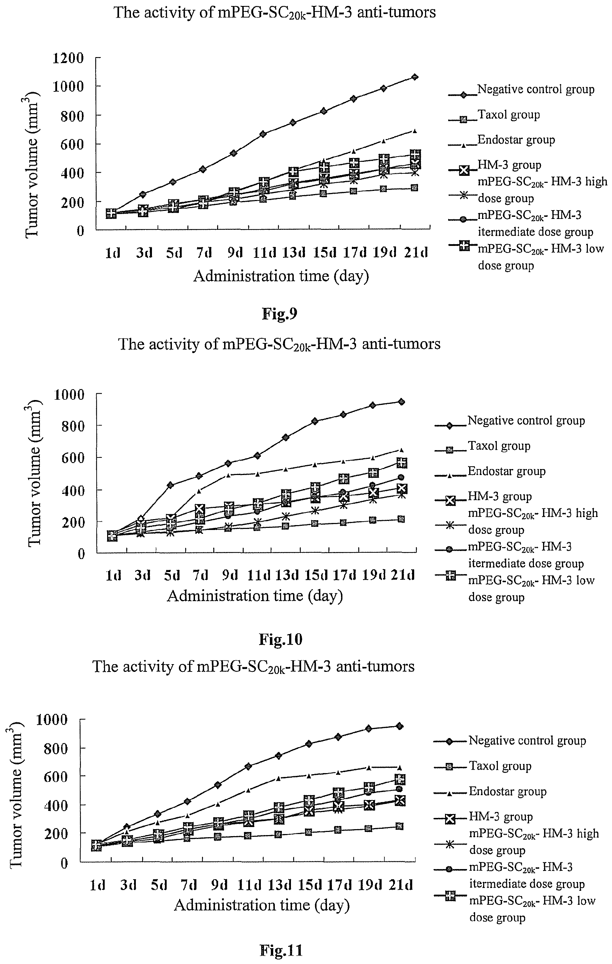

Results: shown in table 8 and figure 9. Taxol group: 10mg/kg, the rate of inhibiting tumor of nude mice with human breast cancer MDA-MB-231 is 73.05%; Endostar group: 2.5mg/kg, the rate of inhibiting tumor of nude mice with human breast cancer MDA-MB-231 is 35.57%; HM-3 group: 1.5mg/kg, the rate of inhibiting tumor of nude mice with human breast cancer MDA-MB-231 is 57.14%; The rates of inhibiting tumor of nude mice with human breast cancer MDA-MB-231 in groups of high, intermediate and low dose of polypeptide are 63.05%, 59.11%, 51.01%, respectively.

The results of experiment of tumor growth inhibition induced by mPEG-SC$_{20k}$-HM-3 on human breast cancer MDA-MB-231 xenograft tumors in nude mice demonstrated that compared with negative control group, mPEG-SC$_{20K}$-HM-3 36.7mg/kg played a significant inhibition role in growth of human breast cancer MDA-MB-231. *$P<0.05$(there is significant difference compared with negative group).

**Example 14 The experiment of tumor growth inhibition induced by mPEG-SC$_{20K}$-HM-3 on human gallbladder cancer GBC-SD xenograft tumors in nude mice**

Human gallbladder cancer GBC-SD cell lines in logarithmic growth phase were picked and made into $5 \times 10^7$/ml cell suspension of under sterile conditions, and then 0.1 ml suspension was inoculated subcutaneously at the right armpit of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein. The mice in the placebo group were injected saline with the same volume, once 1 day; Taxol group: 10mg/kg, once 1 week; Endostar group: 2.5mg/kg, once 1 day; The groups of high, intermediate and low polypeptide: 6, 3, 1.5mg/kg, respectively, once 1 day. After administration, all mice were sacrificed. Stripped the tumors and weighed.

**Table 9 The effect of tumor growth inhibition induced by mPEG-SC$_{20K}$-HM-3 on human gallbladder cancer GBC-SD xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once 1 day | 21.21 | 12 | 20.32 | 12 | 1.061 | - |
| Taxol | 10 | Once 2 days | 20.52 | 8 | 20.43 | 8 | 0.232 | 78.13% |
| Endostar | 2.5 | Once 1 day | 20.68 | 8 | 20.45 | 8 | 0.728 | 31.39% |

(continued)

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| HM-3 | 1.5 | Once 3 days | 21.12 | 8 | 20.92 | 8 | 0.450 | 57.59% |
| mPEG-SC$_{20k}$-HM-3high | 36.7 | Once 3 day | 20.46 | 8 | 20.07 | 8 | 0.409 | 61.45%* |
| mPEG-SC$_{20k}$-HM-3intermediate | 18.75 | Once 3 day | 21.32 | 8 | 20.56 | 8 | 0.524 | 50.59% |
| mPEG-SC$_{20k}$-HM-3low | 9.38 | Once 3 day | 21.05 | 8 | 20.58 | 8 | 0.633 | 40.32% |

Results: shown in table 9 and figure 10. Taxol group: 10mg/kg, the rate of inhibiting tumor of nude mice with human gallbladder cancer GBC-SD is78.13%; Endostar group: 2.5mg/kg, the rate of inhibiting tumor of nude mice with human gallbladder cancer GBC-SD is 31.39%; HM-3 group: 1.5mg/kg, the rate of inhibiting tumor of nude mice with human gallbladder cancer GBC-SD is 57.59%; The rates of inhibiting tumor of nude mice with human gallbladder cancer GBC-SD in groups of high, intermediate and low dose of polypeptide are 61.45%, 50.59%, 40.32%, respectively.

The results of experiment of tumor growth inhibition induced by mPEG-SC$_{20k}$-HM-3 on human gallbladder cancer GBC-SD xenograft tumors in nude mice demonstrated that compared with negative control group, mPEG-SC$_{20K}$-HM-3 36.7mg/kg played a significant inhibition role in growth of human gallbladder cancer GBC-SD. *P<0.05*(there is significant difference compared with negative group).

**Example 15 The experiment of tumor growth inhibition induced by mPEG-SC$_{20K}$-HM-3 on human kidney cancer A498 xenograft tumors in nude mice**

Human kidney cancer A498 cell lines in logarithmic growth phase were picked and made into $5 \times 10^7$/ml cell suspension of under sterile conditions, and then 0.1 ml suspension was inoculated subcutaneously at the right armpit of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein. The mice in the placebo group were injected saline with the same volume, once 1 day; Taxol group: 10mg/kg, once 1 week; Endostar group: 2.5mg/kg, once 1 day; The groups of high, intermediate and low polypeptide: 6, 3, 1.5mg/kg, respectively, once 1 day. After administration, all mice were sacrificed. Stripped the tumors and weighed.

**Table 10 The effect of tumor growth inhibition induced by mPEG-SC$_{20K}$-HM-3 on human kidney cancer A498 xenograft tumors in nude mice**

| Groups | Dose (mg/kg) | Frequency of (administration | Primary weight (g) | Primary numbers | Final weight (g) | Final numbers | Tumor weight | Rate of inhibiting tumors (%) |
|---|---|---|---|---|---|---|---|---|
| Negative control | - | Once 1 day | 21.38 | 10 | 21.11 | 12 | 0.944 | - |
| Taxol | 10 | Once 2 days | 21.23 | 6 | 21.15 | 6 | 0.242 | 74.32% |
| Endostar | 2.5 | Once 1 day | 21.43 | 6 | 21.66 | 6 | 0.656 | 30.51% |
| HM-3 | 1.5 | Once 3 days | 20.47 | 8 | 20.88 | 8 | 0.427 | 54.77% |
| mPEG-SC$_{20k}$-HM-3high | 36.7 | Once 3 days | 20.00 | 6 | 20.23 | 6 | 0.424 | 55.11%* |

(continued)

| Groups | Dose (mg/kg) | Frequency of (administration | Primary weight (g) | Primary numbers | Final weight (g) | Final numbers | Tumor weight | Rate of inhibiting tumors (%) |
|---|---|---|---|---|---|---|---|---|
| mPEG-SC$_{20k}$-HM-3intermediate | 18.75 | Once 3 days | 21.34 | 6 | 20.48 | 6 | 0.501 | 46.95% |
| inPEG-SC$_{20k}$-HM-3low | 9.38 | Once 3 days | 20.35 | 6 | 20.76 | 6 | 0.576 | 39.00% |

Results: shown in table 10 and figure 11. Taxol group: 10mg/kg, the rate of inhibiting tumor of nude mice with human kidney cancer A498 is84.32%; Endostar group: 2.5mg/kg, the rate of inhibiting tumor of nude mice with human kidney cancer A498 is 30.51%; HM-3 group: 1.5mg/kg, the rate of inhibiting tumor of nude mice with human kidney cancer A498 is 54.77%; The rates of inhibiting tumor of nude mice with human kidney cancer A498 in groups of high, intermediate and low dose of polypeptide are55.11%, 46.95%, 39.00%, respectively.

The results of experiment of tumor growth inhibition induced by mPEG-SC$_{20k}$-HM-3 on human kidney cancer A498 xenograft tumors in nude mice demonstrated that compared with negative control group, mPEG-SC$_{20K}$-HM-3 36.7mg/kg played a significant inhibition role in growth of human kidney cancer A498. *$P<0.05$(there is significant difference compared with negative group).

## Example 16 The experiment of tumor growth inhibition induced by mPEG-SC$_{20k}$-HM-3 on human colon cancer HT-29 xenograft tumors in nude mice

Human colon cancer HT-29 cell lines in logarithmic growth phase were picked, and made into $5\times10^7$/ml cell suspension of under sterile conditions, and then 0.1 ml suspension was inoculated subcutaneously at the right armpit of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein. The mice in the placebo group were injected saline with the same volume, once 1 day; Taxol group: 10mg/kg, once 1 week; Endostar group: 2.5mg/kg, once 1 day; The groups of high, intermediate and low polypeptide: 6, 3, 1.5mg/kg, respectively, once 1 day. After administration, all mice were sacrificed. Stripped the tumors and weighed.

**Table 11 The effect of tumor growth inhibition induced by mPEG-SC$_{20k}$-HM-3 on human colon cancer HT-29 xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once 1 day | 23.94 | 12 | 23.67 | 12 | 1.120 | - |
| Taxol | 10 | Once 2 days | 23.43 | 8 | 22.84 | 6 | 0.346 | 69.11% |
| Endostar | 2.5 | Once 1 day | 23.23 | 8 | 22.81 | 8 | 0.745 | 33.48% |
| HM-3 | 1.5 | Once 3 days | 24.11 | 12 | 23.15 | 12 | 0.531 | 52.59% |
| mPEG-SC$_{20k}$-HM-3 high | 36.7 | Once 3 days | 23.45 | 8 | 22.68 | 8 | 0.493 | 55.98%* |
| mPEG-SC$_{20k}$-HM-3 intermediate | 18.75 | Once 3 days | 24.61 | 8 | 25.36 | 8 | 0.611 | 45.45% |
| mPEG-SC$_{20k}$-HM-3 low | 9.38 | Once 3 days | 24.29 | 8 | 23.17 | 8 | 0.705 | 37.05% |

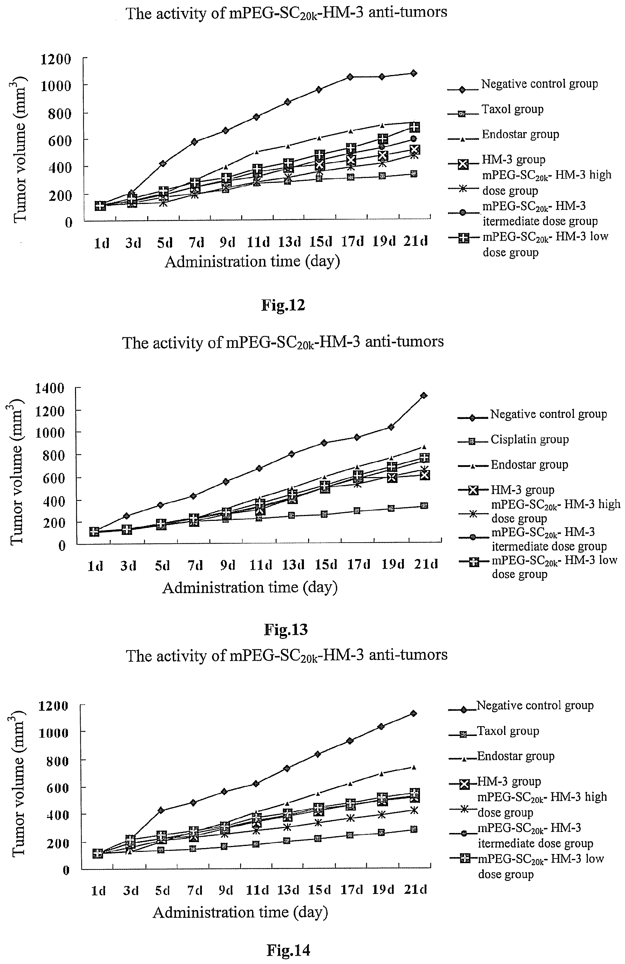

Results: shown in table 11 and figure 12. Taxol group: 10mg/kg, the rate of inhibiting tumor of nude mice with human colon cancer HT-29 is 69.11%; Endostar group: 2.5mg/kg, the rate of inhibiting tumor of nude mice with human colon cancer HT-29 is 33.48%; HM-3 group: 1.5mg/kg, the rate of inhibiting tumor of nude mice with human colon cancer HT-29 is 52.59%; The rates of inhibiting tumor of nude mice with human colon cancer HT-29 in groups of high, intermediate and low dose of polypeptide are 55.98%, 45.45%, 37.05%, respectively.

The results of experiment of tumor growth inhibition induced by mPEG-SC$_{20k}$-HM-3 on human colon cancer HT-29 xenograft tumors in nude mice demonstrated that compared with negative control group, mPEG-SC$_{20k}$-HM-3 36.7mg/kg played a significant inhibition role in growth of human colon cancer HT-29. *P<0.05*(there is significant difference compared with negative group).

## Example 17 The experiment of tumor growth inhibition induced by mPEG-SC$_{20k}$-HM-3 on human ovarian cancer SK-OV-3 xenograft tumors in nude mice

Human ovarian cancer SK-OV-3 cell lines in logarithmic growth phase were picked, and made into $5\times10^7$/ml cell suspension of under sterile conditions, and then 0.1 ml suspension was inoculated subcutaneously at the right armpit of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein. The mice in the placebo group were injected saline with the same volume, once 1 day; Cisplatin group: 10mg/kg, once 1 week; Endostar group: 2.5mg/kg, once 1 day; The groups of high, intermediate and low polypeptide: 6, 3, 1.5mg/kg, respectively, once 1 day. After administration, all mice were sacrificed. Stripped the tumors and weighed.

### Table 12 The effect of tumor growth inhibition induced by mPEG-SC$_{20k}$-HM-3 on human ovarian cancer SK-OV-3 xenograft tumors in nude mice

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once 1 day | 23.51 | 12 | 22.03 | 12 | 1.265 | - |
| Cisplatin | 10 | Twice 1 week | 22.43 | 8 | 22.19 | 6 | 0.302 | 76.13% |
| Endostar | 2.5 | Once 1 day | 22.94 | 8 | 22.74 | 8 | 0.860 | 31.98% |
| HM-3 | 1.5 | Once 3 days | 22.90 | 8 | 22.85 | 8 | 0.639 | 49.49% |
| mPEG-SC$_{20k}$-HM-3 high | 36.7 | Once 3 days | 23.53 | 8 | 22.35 | 8 | 0.627 | 50.40%* |
| mPEG-SC$_{20k}$-HM-3 intermediate | 18.75 | Once 3 days | 23.66 | 8 | 22.29 | 8 | 0.700 | 44.62% |
| mPEG-SC$_{20k}$-HM-3 low | 9.38 | Once 3 days | 22.89 | 8 | 22.74 | 8 | 0.730 | 42.33% |

Results: shown in table 12 and figure 13. Cisplatin group: 10mg/kg, the rate of inhibiting tumor of nude mice with human ovarian cancer SK-OV-3 is 76.13%; Endostar group: 2.5mg/kg, the rate of inhibiting tumor of nude mice with human ovarian cancer SK-OV-3 is 31.98%; HM-3 group: 1.5mg/kg, the rate of inhibiting tumor of nude mice with human ovarian cancer SK-OV-3 is 49.49%; The rates of inhibiting tumor of nude mice with human ovarian cancer SK-OV-3 in groups of high, intermediate and low dose of polypeptide are 50.40%, 44.62%, 42.33%, respectively.

Therefore, the results of experiment of tumor growth inhibition induced by mPEG-SC$_{20k}$-HM-3 on human ovarian cancer SK-OV-3 xenograft tumors in nude mice demonstrated that compared with negative control group, mPEG-SC$_{20K}$-HM-3 36.7mg/kg played a significant inhibition role in growth of human ovarian cancer SK-OV-3. *P<0.05*(there is significant difference compared with negative group).

**Example 18 The experiment of tumor growth inhibition induced by mPEG-SC$_{20K}$-HM-3 on human endometrial cancer HHUA xenograft tumors in nude mice**

Human endometrial cancer HHUA cell lines in logarithmic growth phase were picked, and made into 5x10$^7$/ml cell suspension of under sterile conditions, and then 0.1 ml suspension was inoculated subcutaneously at the right armpit of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein. The mice in the placebo group were injected saline with the same volume, once 1 day; Taxol group: 10mg/kg, once 1 week; Endostar group: 2.5mg/kg, once 1 day; The groups of high, intermediate and low polypeptide: 6, 3, 1.5mg/kg, respectively, once 1 day. After administration, all mice were sacrificed. Stripped the tumors and weighed.

**Table 13 The effect of tumor growth inhibition induced by mPEG-SC$_{20K}$-HM-3 on human endometrial cancer HHUA xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once 1 day | 24.51 | 12 | 23.03 | 12 | 1.208 | - |
| Taxol | 10 | Twicw 1 week | 23.43 | 8 | 22.19 | 6 | 0.295 | 75.55% |
| Endostar | 2.5 | Once 1 day | 22.98 | 8 | 22.74 | 8 | 0.785 | 34.98% |
| HM-3 | 1.5 | Once 3 days | 22.94 | 8 | 22.85 | 8 | 0.550 | 54.49% |
| mPEG-SC$_{20k}$-HM-3 high | 36.7 | Once 3 days | 23.98 | 8 | 22.39 | 8 | 0.453 | 62.47%* |
| mPEG-SC$_{20k}$-HM-3 itermediate | 18.75 | Once 3 days | 23.76 | 8 | 22.37 | 8 | 0.560 | 53.65% |
| mPEG-SC$_{20k}$-HM-3 low | 9.38 | Once 3 days | 22.89 | 8 | 22.66 | 8 | 0.587 | 51.38% |

Results: shown in table 13 and figure 14. Taxol group: 10mg/kg, the rate of inhibiting tumor of nude mice with human endometrial cancer HHUA is 75.55%; Endostar group: 2.5mg/kg, the rate of inhibiting tumor of nude mice with human endometrial cancer HHUA is 34.98%; HM-3 group: 1.5mg/kg, the rate of inhibiting tumor of nude mice with human endometrial cancer HHUA is 54.49%; The rates of inhibiting tumor of nude mice with human endometrial cancer HHUA in groups of high, intermediate and low dose of polypeptide are 62.47%, 53.65%, 51.38%, respectively.

Therefore, the results of experiment of tumor growth inhibition induced by mPEG-SC$_{20k}$-HM-3 on human endometrial cancer HHUA xenograft tumors in nude mice demonstrated that compared with negative control group, mPEG-SC$_{20K}$-HM-3 36.7mg/kg played a significant inhibition role in growth of human endometrial cancer HHUA. *P<0.05*(there is significant difference compared with negative group).

**Example 19 The experiment of tumor growth inhibition induced by mPEG-SC$_{20K}$-HM-3 on human cervical cancer HeLa xenograft tumors in nude mice**

Human cervical cancer HeLa cell lines in logarithmic growth phase were picked, and made into $5\times10^7$/ml cell suspension of under sterile conditions, and then 0.1 ml suspension was inoculated subcutaneously at the right armpit of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein. The mice in the placebo group were injected saline with the same volume, once 1 day; Taxol group: 10mg/kg, once 1 week; Endostar group: 2.5mg/kg, once 1 day; The groups of high, intermediate and low polypeptide: 6, 3, 1.5mg/kg, respectively, once 1 day. After administration, all mice were sacrificed. Stripped the tumors and weighed.

**Table 14 The effect of tumor growth inhibition induced by mPEG-SC$_{20K}$-HM-3 on human cervical cancer HeLa xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once 1 day | 23.11 | 8 | 23.35 | 8 | 1.236 | - |
| Taxol | 10 | Once 2 days | 24.36 | 6 | 23.47 | 6 | 0.428 | 65.37% |
| Endostar | 2.5 | Once 1 day | 23.54 | 6 | 23.28 | 6 | 0.796 | 35.57% |
| HM-3 | 1.5 | Once 3 days | 24.11 | 6 | 23.86 | 6 | 0.528 | 57.25% |
| mPEG-SC$_{20k}$-HM-3 high | 36.7 | Once 3 days | 23.76 | 6 | 23.05 | 6 | 0.222 | 82.07%* |
| mPEG-SC$_{20k}$-HM-3 itermediate | 18.75 | Once 3 days | 24.80 | 6 | 24.43 | 6 | 0.320 | 74.11% |
| mPEG-SC$_{20k}$-HM-3 low | 9.38 | Once 3 days | 24.10 | 6 | 23.85 | 6 | 0.453 | 63.32% |

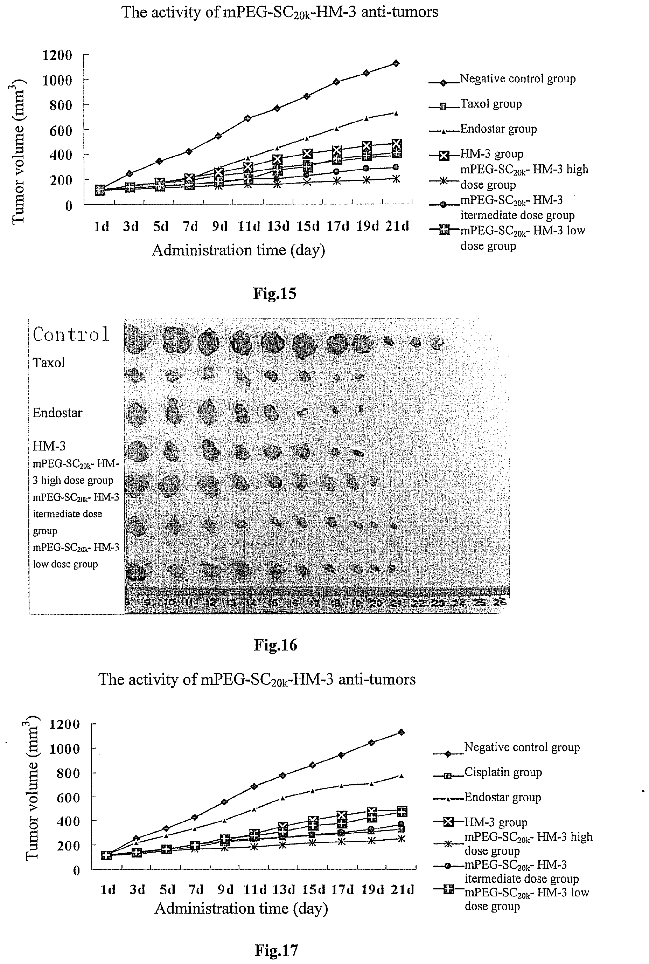

Results: shown in table 14 and figure 15,16. Taxol group: 10mg/kg, the rate of inhibiting tumor of nude mice with human cervical cancer HeLa is 65.37%; Endostar group: 2.5mg/kg, the rate of inhibiting tumor of nude mice with human cervical cancer HeLa is 35.57%; HM-3 group: 1.5mg/kg, the rate of inhibiting tumor of nude mice with human cervical cancer HeLa is 57.25%; The rates of inhibiting tumor of nude mice with human cervical cancer HeLa in groups of high, intermediate and low dose of polypeptide are 82.07%, 74.11%, 63.32%, respectively.

Therefore, the results of experiment of tumor growth inhibition induced by mPEG-SC$_{20k}$-HM-3 on human cervical cancer HeLa xenograft tumors in nude mice demonstrated that compared with negative control group, mPEG-SC$_{20K}$-HM-3 36.7mg/kg played a significant inhibition role in growth of human cervical cancer HeLa. *P<0.05*(there is significant difference compared with negative group)

**Example 20 The experiment of tumor growth inhibition induced by mPEG-SC$_{20K}$-HM-3 on human prostate cancer DU-145 xenograft tumors in nude mice**

Human prostate cancer DU-145 cell lines in logarithmic growth phase were picked, and made into $5 \times 10^7$/ml cell suspension of under sterile conditions, and then 0.1 ml suspension was inoculated subcutaneously at the right armpit of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein. The mice in the placebo group were injected saline with the same volume, once 1 day; Cisplatin group: 10mg/kg, once 1 week; Endostar group: 2.5mg/kg, once 1 day; The groups of high, intermediate and low polypeptide: 6, 3, 1.5mg/kg, respectively, once 1 day. After administration, all mice were sacrificed. Stripped the tumors and weighed.

**Table 15 The effect of tumor growth inhibition induced by mPEG-SC$_{20K}$-HM-3 on human prostate cancer DU-145 xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once 1 day | 24.12 | 12 | 23.65 | 12 | 1.854 | - |
| Taxol | 10 | Twice a week | 24.57 | 8 | 23.81 | 6 | 0.545 | 70.60% |
| Endostar | 2.5 | Once 1 day | 23.08 | 8 | 23.25 | 8 | 1.274 | 31.25% |

(continued)

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| HM-3 | 1.5 | Once 3 days | 24.77 | 8 | 23.64 | 8 | 0.809 | 56.36% |
| mPEG-SC$_{20k}$-HM-3 high | 36.7 | Once 3 days | 23.26 | 8 | 23.08 | 8 | 0.417 | 77.46%* |
| mPEG-SC$_{20k}$-HM-3 itermediate | 18.75 | Once 3 days | 24.38 | 8 | 23.61 | 8 | 0.603 | 67.48% |
| mPEG-SC$_{20k}$-HM-3 low | 9.38 | Once 3 days | 24.41 | 8 | 23.37 | 8 | 0.781 | 57.87% |

Results: shown in table 15 and figure 17. Cisplatin group: 10mg/kg, the rate of inhibiting tumor of nude mice with human prostate cancer DU-145 is 70.60%; Endostar group: 2.5mg/kg, the rate of inhibiting tumor of nude mice with human prostate cancer DU-145 is 31.25%; HM-3 group: 1.5mg/kg, the rate of inhibiting tumor of nude mice with human prostate cancer DU-145 is 56.36%; The rates of inhibiting tumor of nude mice with human prostate cancer DU-145 in groups of high, intermediate and low dose of polypeptide are 77.46%, 67.48%, 57.87%, respectively.

Therefore, the results of experiment of tumor growth inhibition induced by mPEG-SC$_{20k}$-HM-3 on human prostate cancer DU-145 xenograft tumors in nude mice demonstrated that compared with negative control group, mPEG-SC$_{20K}$-HM-3 36.7mg/kg played a significant inhibition role in growth of human prostate cancer DU-145. *$P<0.05$(there is significant difference compared with negative group).

**Example 21 The experiment of tumor growth inhibition induced by mPEG-SC$_{20K}$-HM-3 on human bladder cancer HT1376 xenograft tumors in nude mice**

Human bladder cancer HT1376 cell lines in logarithmic growth phase were picked, and made into $5\times10^7$/ml cell suspension of under sterile conditions, and then 0.1 ml suspension was inoculated subcutaneously at the right armpit of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein. The mice in the placebo group were injected saline with the same volume, once 1 day; Taxol group: 10mg/kg, once 1 week; Endostar group: 2.5mg/kg, once 1 day; The groups of high, intermediate and low polypeptide: 6, 3, 1.5mg/kg, respectively, once 1 day. After administration, all mice were sacrificed. Stripped the tumors and weighed.

**Table 16 The effect of tumor growth inhibition induced by mPEG-SC$_{20K}$-HM-3 on human bladder cancer HT1376 xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once1 day | 21.71 | 10 | 21.32 | 10 | 1.018 | - |
| Taxol | 10 | Once 2 days | 21.39 | 6 | 21.65 | 5 | 0.327 | 67.88% |
| Endostar | 2.5 | Once 1 day | 21.52 | 6 | 21.36 | 6 | 0.698 | 31.43% |
| HM-3 | 1.5 | Once 3 days | 22.84 | 6 | 22.30 | 6 | 0.504 | 50.49% |
| mPEG-SC$_{20k}$-HM-3 high | 36.7 | Once 3 days | 22.27 | 6 | 21.59 | 6 | 0.372 | 63.42%* |
| mPEG-SC$_{20k}$-HM-3 itermediate | 18.75 | Once 3 days | 21.58 | 6 | 21.49 | 6 | 0.466 | 54.24% |

(continued)

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| mPEG-SC$_{20k}$-HM-3 low | 9.38 | Once 3 days | 22.76 | 6 | 22.23 | 6 | 0.546 | 46.39% |

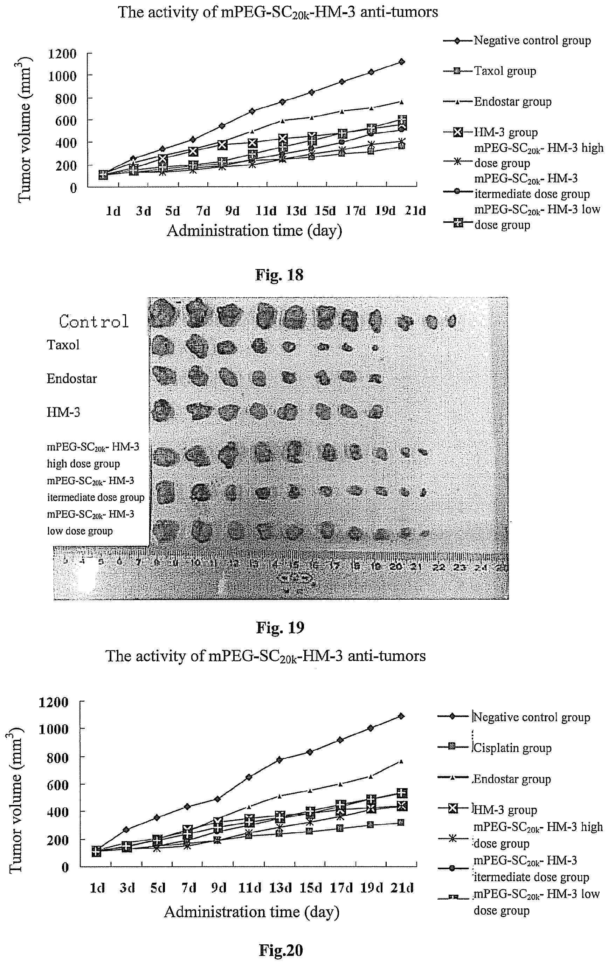

Results: shown in table 16 and figure 18,19. Taxol group: 10mg/kg, the rate of inhibiting tumor of nude mice with human bladder cancer HT1376 is 67.88%; Endostar group: 2.5mg/kg, the rate of inhibiting tumor of nude mice with human bladder cancer HT1376 is 31.43%; HM-3 group: 1.5mg/kg, the rate of inhibiting tumor of nude mice with human bladder cancer HT1376 is 50.49%; The rates of inhibiting tumor of nude mice with human bladder cancer HT1376 in groups of high, intermediate and low dose of polypeptide are 63.42%, 54.24%, 46.39%, respectively.

Therefore, the results of experiment of tumor growth inhibition induced by mPEG-SC$_{20k}$-HM-3 on human bladder cancer HT1376 xenograft tumors in nude mice demonstrated that compared with negative control group, mPEG-SC$_{20k}$-HM-3 36.7mg/kg played a significant inhibition role in growth of human bladder cancer HT1376. *P<0.05(there is significant difference compared with negative group).

**Example 22 The experiment of tumor growth inhibition induced by mPEG-SC$_{20K}$-HM-3 on human testicular cancer 5637 xenograft tumors in nude mice**

Human testicular cancer 5637 cell lines in logarithmic growth phase were picked, and made into $5 \times 10^7$/ml cell suspension of under sterile conditions, and then 0.1 ml suspension was inoculated subcutaneously at the right armpit of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein. The mice in the placebo group were injected saline with the same volume, once 1 day; Cisplatin group: 10mg/kg, once 1 week; Endostar group: 2.5mg/kg, once 1 day; The groups of high, intermediate and low polypeptide: 6, 3, 1.5mg/kg, respectively, once 1 day. After administration, all mice were sacrificed. Stripped the tumors and weighed.

**Table 17 The effect of tumor growth inhibition induced by mPEG-SC$_{20K}$-HM-3 on human testicular cancer 5637 xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once 1 day | 24.08 | 10 | 23.91 | 10 | 2.015 | - |
| Taxol | 10 | Twice 1 week | 24.73 | 6 | 24.46 | 6 | 0.590 | 70.74% |
| Endostar | 2.5 | Once 1 day | 25.07 | 6 | 24.89 | 6 | 1.410 | 30.02% |
| HM-3 | 1.5 | Once 3 days | 25.66 | 6 | 25.15 | 6 | 0.82 | 59.40% |
| mPEG-SC$_{20k}$-HM-3 high | 36.7 | Once 3 days | 25.45 | 6 | 24.18 | 6 | 0.802 | 60.20%* |
| mPEG-SC$_{20k}$-HM-3 itermediate | 18.75 | Once 3 days | 24.24 | 6 | 24.19 | 6 | 0.981 | 51.32% |
| mPEG-SC$_{20k}$-HM-3 low | 9.38 | Once 3 days | 25.74 | 6 | 25.18 | 6 | 0.988 | 50.99% |

Results: shown in table 17 and figure20. Cisplatin group: 10mg/kg, the rate of inhibiting tumor of nude mice with human testicular cancer 5637 is70.74%; Endostar group: 2.5mg/kg, the rate of inhibiting tumor of nude mice with human testicular cancer 5637 is 30.02%; HM-3 group: 1.5mg/kg, the rate of inhibiting tumor of nude mice with human testicular cancer 5637 is 39.40%; The rates of inhibiting tumor of nude mice with human testicular cancer 5637 in groups of high, intermediate and low dose of polypeptide are 60.20%, 51.32%, 50.99%, respectively.

Therefore, the results of experiment of tumor growth inhibition induced by mPEG-SC$_{20k}$-HM-3 on human testicular cancer 5637 xenograft tumors in nude mice demonstrated that compared with negative control group, mPEG-SC$_{20K}$-HM-3 36.7mg/kg played a significant inhibition role in growth of human testicular cancer 5637. *P<0.05*(there is significant difference compared with negative group).

**Example 25 The experiment of tumor growth inhibition induced by mPEG-SC$_{20K}$-HM-3 on sarcoma HT-1080 xenograft tumors in nude mice**

Sarcoma HT-1080 cell lines in logarithmic growth phase were picked, and made into $5 \times 10^7$/ml cell suspension of under sterile conditions, and then 0.1 ml suspension was inoculated subcutaneously at the right armpit of nude mice. Tumor diameter was measured with a vernier caliper. The animals which had tumors with a size of 100-200mm$^3$ were randomly divided into groups. The effect of polypeptide on anti-tumor was dynamically observed through the method of measuring tumor diameter. The times of measuring tumor diameter were once 2 days, and meantime, weighed mice. The mice in experimental group were infused polypeptide through tail vein. The mice in the placebo group were injected saline with the same volume, once 1 day; Cyclophosphamide group: 15mg/kg, once 1 week; Polypeptide group: 3mg/kg, once 1 day. After administration, all mice were sacrificed. Stripped the tumors and weighed.

**Table 18 The effect of tumor growth inhibition induced by mPEG-SC$_{20K}$-HM-3 on sarcoma HT-1080 xenograft tumors in nude mice**

| Groups | Dose (mg/kg) | Frequency of administration | Primary weight (g) | Primary numbers | Final weight (g) | Final numbers | Tumor weight (g) | Rate of inhibiting tumors (%) |
|---|---|---|---|---|---|---|---|---|
| Negative control | - | Once 1 day | 23.13 | 8 | 22.81 | 8 | 1.938 | - |
| Cyclophosphamide | 15 | Once 2 days | 24.09 | 8 | 23.85 | 8 | 0.501 | 74.15% |
| HM-3 | 1.5 | Once 3 days | 24.20 | 8 | 23.89 | 8 | 0.790 | 59.24% |
| mPEG-SC$_{20k}$-HM-3 | 36.7 | Once 3 days | 23.57 | 8 | 23.26 | 8 | 0.678 | 65.04%* |

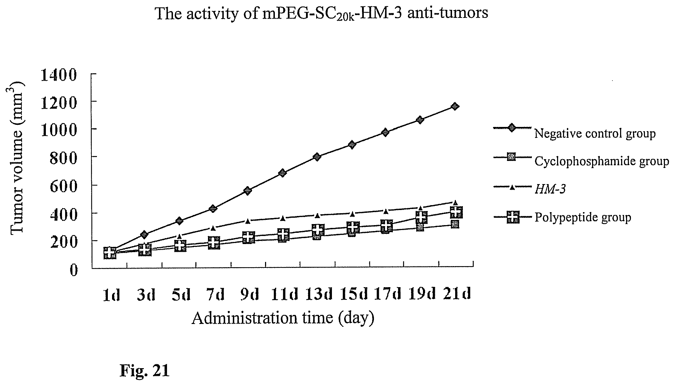

Results: shown in table 18 and figure21. Cyclophosphamide group: 10mg/kg, the rate of inhibiting tumor of nude mice with sarcoma HT-1080 is74.15%; HM-3 group: 1.5mg/kg, the rate of inhibiting tumor of nude mice with sarcoma HT-1080 is 59.24%; The rate of inhibiting tumor of nude mice with human sarcoma HT-1080 in polypeptide group is 65.04%.

Therefore, the results of experiment of tumor growth inhibition induced by mPEG-SC$_{20k}$-HM-3 on sarcoma HT-1080 xenograft tumors in nude mice demonstrated that compared with negative control group, mPEG-SC$_{20K}$-HM-3 36.7mg/kg played a significant inhibition role in growth of sarcoma HT-1080. *$P<0.05$(there is significant difference compared with negative group)

## Claims

1. An integrin inhibitor modified by polyethylene glycol for use in the treatment of a tumor originated from stomach, or head and neck, said integrin inhibitor designated as HM-3 and having the sequence mPEG-Ile-Val-Arg-Arg-Ala-Asp-Arg-Ala-Ala-Val-Pro-Gly-Gly-Gly-Gly-Arg-Gly-Asp, wherein the molecular weight of mPEG-SC is 500-20000.

2. The integrin inhibitor for use of claim 1, wherein the molecular weight of mPEG-SC is 20000.

3. The integrin inhibitor for use of claim 2, wherein the frequency of administration is once every 2-3 days.

## Patentansprüche

1. Integrininhibitor, der durch Polyethylenglykol modifiziert ist, zur Verwendung bei der Behandlung eines Tumors, der aus dem Magen oder Kopf- und Hals-Bereich stammt, wobei der Integrininhibitor als HM-3 bezeichnet wird und die Sequenz mPEG-Ile-Val-Arg-Arg-Ala-Asp-Arg-Ala-Ala-Val-Pro-Gly-Gly-Gly-Gly-Arg-Gly-Asp hat, wobei das Molekulargewicht von mPEG-SC 500-20000 ist.

2. Integrininhibitor zur Verwendung nach Anspruch 1, wobei das Molekulargewicht von mPEG-SC 20000 ist.

3. Integrininhibitor zur Verwendung nach Anspruch 2, wobei die Häufigkeit der Verabreichung einmal alle 2-3 Tage ist.

## Revendications

1. Inhibiteur d'intégrine modifié par du polyéthylène glycol pour une utilisation dans le traitement d'une tumeur provenant de l'estomac, ou de la tête et du cou, ledit inhibiteur d'intégrine désigné comme étant HM-3 et ayant la séquence mPEG-Ile-Val-Arg-Arg-Ala-Asp-Arg-Ala-Ala-Val-Pro-Gly-Gly-Gly-Gly-Arg-Gly-Asp, dans lequel la masse moléculaire de mPEG-SC est de 500 à 20 000.

2. Inhibiteur d'intégrine pour une utilisation selon la revendication 1, dans lequel la masse moléculaire de mPEG-SC est de 20 000.

3. Inhibiteur d'intégrine pour une utilisation selon la revendication 2, dans lequel la fréquence d'administration est d'une fois tous les 2 à 3 jours.

# Illustrations of instruction

**Fig. 1**

**Fig.2**

The activity of mPEG-SC20k-HM-3 anti-tumors

**Fig.3**

The activity of mPEG-SC20k-HM-3 anti-tumors

**Fig.4**

The activity of mPEG-SC20k-HM-3 anti-tumors

**Fig.5**

The activity of mPEG-SC$_{20k}$-HM-3 anti-tumors

**Fig.6**

The activity of mPEG-SC$_{20k}$-HM-3 anti-tumors

**Fig.7**

The activity of mPEG-SC$_{20k}$-HM-3 anti-tumors

**Fig.8**

The activity of mPEG-SC$_{20k}$-HM-3 anti-tumors

**Fig.9**

The activity of mPEG-SC$_{20k}$-HM-3 anti-tumors

**Fig.10**

The activity of mPEG-SC$_{20k}$-HM-3 anti-tumors

**Fig.11**

The activity of mPEG-SC20k-HM-3 anti-tumors

Fig.12

The activity of mPEG-SC20k-HM-3 anti-tumors

Fig.13

The activity of mPEG-SC20k-HM-3 anti-tumors

Fig.14

The activity of mPEG-SC$_{20k}$-HM-3 anti-tumors

**Fig.15**

**Fig.16**

The activity of mPEG-SC$_{20k}$-HM-3 anti-tumors

**Fig.17**

The activity of mPEG-SC$_{20k}$-HM-3 anti-tumors

**Fig. 18**

**Fig. 19**

The activity of mPEG-SC$_{20k}$-HM-3 anti-tumors

**Fig.20**

The activity of mPEG-SC$_{20k}$-HM-3 anti-tumors

Fig. 21

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102145161 A
- CN 2012084788 W

- CN 201110370529

**Non-patent literature cited in the description**

- Molecular Cloning

SEQUENCE LISTING

```
<110> Xu, Hanmei
<120> Polyethylene Glycol-modified Integrin Blocker HM-3 and Use Thereof
<130> X30346WOEP
<140> PCT/CN2012/084788
<141> 2012-12-17
<150> CN201110370529.9
<151> 2011-11-21
<160> 5
<210> 1
<211> 18
<212> PRT
<213> Artificial Sequence
<220>
<223> integrin antagonist peptide
<400> 1
```

```
        Ile Val Arg Arg Ala Asp Arg Ala Ala Val Pro Gly Gly Gly Gly Arg
         1               5                   10                  15
        Gly Asp
```

```
                              <210>  2
                              <211>  11
                              <212>  PRT
                              <213>  homo sapiens
                              <400>  2
```

```
        Ile Val Arg Arg Ala Asp Arg Ala Ala Val Pro
         1               5                   10
```

```
<210> 3
<211> 7
<212> PRT
<213> Artificial Sequence
<220>
<223> Partial sequence of the integrin antagonist peptide of SEQ ID No.1
<400> 3
```

```
                Gly Gly Gly Gly Arg Gly Asp
                 1               5
```

```
<210> 4
<211> 3
<212> PRT
<213> homo sapiens
```

<400> 4

```
                              Arg Gly Asp
                               1
```

<210> 5
<211> 18
<212> PRT
<213> Artificial Sequence
<220>
<223> integrin inhibitor peptide has identical amino sequences with SEQ
NO.1 and N-terminally modified with mPEG-SC
<400> 5

```
        Ile Val Arg Arg Ala Asp Arg Ala Ala Val Pro Gly Gly Gly Gly Arg
         1                   5                   10                  15
        Gly Asp
```